# FASCICULE DE BREVET EUROPEEN

(11) **EP 1 994 061 B1**
(45) Date de publication et mention de la délivrance du brevet: **15.03.2017**
(21) Numéro de dépôt: 07730987.0
(22) Date de dépôt: 15.02.2007
(51) Int. Cl.: C08B 30/18, A61M 1/28, C12P 19/20, A61K 31/195, A61K 31/718

(54) **POLYMERES SOLUBLES DE GLUCOSE HAUTEMENT BRANCHES POUR LA NUTRITION ENTERALE, PARENTERALE ET POUR LA DIALYSE PERITONEALE**
LÖSLICHE HOCHVERZWEIGTE GLUCOSEPOLYMERE FÜR ENTERALE UND PARENTERALE ERNÄHRUNG SOWIE FÜR PERITONEALDIALYSE
SOLUBLE, HIGHLY BRANCHED GLUCOSE POLYMERS FOR ENTERAL AND PARENTERAL NUTRITION AND FOR PERITONEAL DIALYSIS

(30) Priorité: 28.02.2006 FR 0601778
(43) Date de publication de la demande: 26.11.2008
(73) Titulaire: Roquette Frères, 62136 Lestrem (FR)
(72) Inventeur: DEREMAUX, Laëtitia, F-59800 Lille (FR); PETITJEAN, Carole, F-59520 Marquette Lez Lille (FR); WILLS, Daniel, F-59190 Morbecque (FR)
(74) Mandataire: Cabinet Plasseraud
(86) Numéro de dépôt international: PCT/FR2007/000276
(87) Numéro de publication internationale: WO 2007/099212

(56) Documents cités:
- EP-A1- 1 006 128
- EP-A2- 1 369 432
- EP-A2- 1 548 033
- WO-A-2005/083103

## Description

L'invention a pour objet des polymères solubles de glucose hautement branchés, obtenus par traitement enzymatique d'amidon, ayant une teneur en sucres réducteurs inférieure à 3,5 %, de préférence inférieure à 2,5 %, plus préférentiellement encore inférieure à 1 % et présentant un taux de liaisons glucosidiques α-1,6 compris entre 20 et 30 %, et une masse moléculaire moyenne en poids (Mw) choisie dans une fourchette très étroite comprise entre 20.10³ et moins de 30.10³ daltons.

Les polymères solubles de glucose hautement branchés de la présente invention présentent en outre une faible osmolalité, déterminée selon un test A, d'une valeur inférieure à 25 mOsm/kg, de préférence comprise entre 5 et 20 mOsm/kg.

Ces polymères peuvent être également hydrogénés ou isomérisés pour accroître leur stabilité, notamment thermique.

Ces polymères solubles de glucose hautement branchés présentent par ailleurs une faible viscosité et une absence de rétrogradation, même après stockage à froid après de longues périodes.

L'invention concerne également un procédé de fabrication de tels polymères solubles de glucose hautement branchés.

L'invention a en outre pour objet des compositions comprenant de tels polymères solubles de glucose hautement branchés. Ces compositions sont utilisables dans de nombreuses applications industrielles, notamment dans les industries alimentaires et pharmaceutiques.

L'adjectif « soluble » utilisé en relation avec les polymères de glucose de la présente invention signifie que ces polymères sont solubles dans l'eau.

Les polymères de glucose classiquement fabriqués industriellement sont préparés par hydrolyse d'amidons naturels ou hybrides et de leurs dérivés.

Les hydrolysats d'amidon classiques sont ainsi produits par hydrolyse acide ou enzymatique d'amidon de céréales ou de tubercules. Ils sont en fait un mélange de glucose et de polymères du glucose, de poids moléculaires extrêmement variés.

Ces hydrolysats d'amidon (dextrines, maltodextrines...) produits dans l'industrie (avec un certain Degré de Polymérisation ou DP moyen) comprennent une large distribution de saccharides contenant à la fois des structures linéaires (liaisons glucosidiques α-1,4) et branchées (liaisons glucosidiques α-1,6).

Ces hydrolysats d'amidon, et notamment les maltodextrines, sont généralement utilisés comme transporteur ou agent de charge, agent texturant, support d'atomisation, agent de remplacement de matières grasses, agent filmogène, agent de contrôle de congélation, agent anti-cristallisant, ou pour leur apport nutritionnel.

Il est connu de l'homme du métier que la composition saccharidique des maltodextrines détermine à la fois leurs propriétés physiques et biologiques.

C'est ainsi que leur hygroscopicité, leur fermentescibilité, leur viscosité, leur caractère édulcorant, leur stabilité, leur caractère gélifiant et leur osmolalité sont des critères classiquement appréciés et choisis selon leurs différents domaines d'application.

Les connaissances de base du comportement physico-chimique de ces saccharides conduisent ainsi à les intégrer par exemple dans les solutions pour dialyse péritonéale, les liquides parentéraux et entéraux ou dans des aliments pour diabétiques.

De ce fait, pour ces différentes applications, diverses propriétés physiques et biologiques sont requises.

Il est par exemple connu que le taux d'absorption de ces saccharides est déterminé par la vitesse de vidange gastrique et par le taux d'adsorption intestinal, dont le contrôle est assuré par l'osmolalité desdits saccharides.

Au niveau intestinal, les maltodextrines sont hydrolysées par l'α-amylase pancréatique, ce qui conduit à réduire leur taille jusqu'aux dextrines limites, puis un certain nombre d'enzymes liées à la muqueuse intestinale, telles que la maltase, la sucrase et l'α-dextrinase, continuent à hydrolyser les saccharides linéaires et branchés en glucose.

Si le glucose, le maltose et le maltotriose passent facilement la barrière intestinale (diffusion passive), il n'en est pas de même pour les oligosaccharides supérieurs. En outre, les oligosaccharides linéaires sont absorbés plus vite que les oligosaccharides branchés.

Les bactéries du colon vont par la suite fermenter tous les hydrates de carbone qui n'ont pas traversé la paroi de l'intestin grêle. Une fermentation excessive par ces bactéries amène alors souvent des désordres intestinaux tels les crampes et les flatulences.

On sait également que l'osmolalité influence le taux d'absorption et de sécrétion d'eau dans l'intestin grêle. Plus l'osmolalité d'un composé est élevée, plus celui-ci induit une entrée de fluide dans l'intestin et conduit à des dérèglements de l'intestin (diarrhée osmotique), avec perte concomitante de fluides et d'électrolytes.

L'osmolalité d'une solution est définie comme la quantité de moles dissoutes par kg d'eau, impliquant qu'à la même concentration en poids sec, l'osmolalité d'une maltodextrine classique augmente avec l'abaissement de son DP.

Une osmolalité élevée signifie que les substances de bas poids moléculaire se lient à l'eau, ce qui rend difficile le transport de l'eau et des nutriments au travers de la paroi intestinale. L'osmolalité du sang est d'environ 300 mOsm/kg, et dans le but de faciliter le transport des nutriments, il est souhaitable que l'osmolalité de la substance soit notablement en dessous de cette valeur.

Une dextrine selon WO 95/22.562, d'un poids moléculaire moyen d'environ 720.000 daltons et d'un degré de branchement de 4 % environ, est décrite comme présentant une osmolalité de 20 mOsm/kg.

Ces dextrines sont préparées par traitement acide de l'amidon natif, plus particulièrement de la fécule de pomme de terre, dans des conditions de température élevées, i.e. 110 à 140 °C et dans un temps de réaction de 1 à 15 heures, ce qui conduit à la formation de branchements en 1,6 qui correspondent à la fois à des liaisons glucosidiques α-1,6 et β-1,6.

Les liaisons glucosidiques P atypiques ne sont pas hydrolysées par les systèmes enzymatiques de l'intestin, et conduisent à l'accumulation de résidus non digestibles que certaines bactéries indésirables du colon vont assimiler.

Dans le domaine des solutions parentérales et entérales, des solutions nutritives sont conçues pour maintenir en bonne santé un patient et pour lui fournir les nutriments quand il ne peut être alimenté par les voies digestives naturelles.

Lorsque les solutions sont directement apportées par voie veineuse, elles doivent être isotoniques et l'apport en glucose est ainsi limité.

Pour fournir une énergie journalière de 10.000 kJ, il est décrit dans un article de Food Science Technology de 1999, pp 345-355 par MARCHAL *et al.,* qu'il serait nécessaire de perfuser 14 litres de solution isotonique de glucose (5 % poids/volume de glucose), ce qui dépasse largement les capacités humaines.

L'apport de solutions plus concentrées en glucose ou fructose (10 à 20 % poids/volume) est possible, mais pas pour de longues périodes et à condition d'effectuer la perfusion dans de gros vaisseaux, veine sous-claviaire par exemple.

Il est aussi possible d'administrer des saccharides linéaires avec un DP compris entre 2 et 5, puisque ces saccharides sont hydrolysés par des maltases dans le rein et le glucose libéré est alors réabsorbé. C'est ainsi que l'utilisation de courts oligosaccharides linéaires permet d'apporter suffisamment d'énergie dans une solution isotonique, sans sur-hydrater le patient.

Par ailleurs, les oligosaccharides linéaires d'un DP inférieur à 7 étant stables en solution sur de longues périodes de temps, il est classiquement choisi de faire varier le DP entre 2 et 7 pour permettre d'apporter constamment aux patients, sur ces longues périodes, toute l'énergie nécessaire.

Mais cette solution n'est pas totalement satisfaisante, et elle n'envisage que l'exploitation de structures glucosidiques linéaires.

Quant à la nutrition entérale, elle concerne des boissons qui peuvent être ingérées oralement ou bien être administrées par voie tubulaire dans l'estomac ou l'intestin grêle.

Pour ces fluides entéraux, le problème majeur est la diarrhée, due à une trop forte osmolalité.

De manière classique, pour remédier à ce problème, on utilise des maltodextrines renfermant un mélange complexe de saccharides linéaires et branchés, avec un équivalent dextrose (DE) de 10 à 20. Ces maltodextrines ne donnent toutefois pas entière satisfaction.

Les spécialistes de la nutrition entérale et parentérale recherchent la solution de ces problèmes techniques dans l'élaboration de structures ramifiées dans des produits dérivés de l'amidon.

L'amylopectine, principal constituant de l'amidon, s'organise autour de liaisons α-1,4 linéaires et de liaisons α-1,6 qui constituent des points de ramification. La connaissance des microstructures a mis en évidence que ces deux types de liaisons ne se trouvent pas réparties de manière uniforme, mais que des zones très denses en liaisons α-1,6 y côtoient des zones constituées uniquement de liaisons α-1,4.

Il a été proposé, dans le brevet US 4 840 807, ou la demande de brevet JP 11/187 708, d'extraire les seules zones denses en liaisons α-1,6, comme source de glucides à absorption lente, au sens où les liaisons α-1,6 sont plus lentes à dégrader que les liaisons α-1,4.

Deux familles de produits ont ainsi été développées. La première concerne les dextrines limites préparées par la dégradation des zones à liaisons α-1,4 par une α-amylase seule, et les dextrines préparées par la dégradation des zones à liaisons α-1,4 par l'action simultanée d'une α-amylase et d'une α-amylase.

La résistance de ces dextrines limites aux enzymes digestives humaines permet de les utiliser pour réguler la digestion, mais également pour contrôler la glycémie (application pour l'alimentation des diabétiques). Cet effet est attribué à un retard de la vitesse d'absorption digestive.

Cependant, ces composés ont l'inconvénient de présenter un poids moléculaire très faible, ce qui en limite l'exploitation dans les autres domaines d'application où il faut disposer de produits présentant une certaine viscosité.

Le brevet EP 207.676 enseigne que pour un usage en dialyse péritonéale continue et ambulatoire, on préfère des hydrolysats d'amidon formant des solutions limpides et incolores à 10 % dans l'eau, ayant un Mw de 5.10³ à 1.10⁶ daltons et un indice de polymolécularité ou Ip faible.

Cela se traduit par des compositions qui renferment majoritairement des polymères de glucose de haut poids moléculaire compris entre 5.10³ et 5.10⁵ daltons, qui ne renferment pas ou très peu de glucose ou d'oligosaccharides de DP inférieur ou égal à 3, et pas ou très peu de polymères de glucose de Mw supérieur à 1.10⁶ daltons.

En effet, pour cette application, les monomères ou polymères de faible poids moléculaire traversant rapidement la paroi péritonéale sont sans intérêt pour la création d'un gradient de pression osmotique durable, et d'autre part, les polymères de très haut poids moléculaire, supérieur à 1.106 daltons et qui sont dénués de tout pouvoir osmotique, sont à éviter et même à proscrire puisqu'ils risquent de rétrograder et de précipiter dans l'organisme.

La dialyse péritonéale consiste à introduire une solution de dialyse dans la cavité péritonéale au moyen d'un cathéter. Au bout d'un certain temps, un échange de solutés se produit entre le sang et le dialysat. L'utilisation d'un agent osmotique adéquat permet le drainage de l'eau excédentaire, du sang vers le dialysat et de pallier ainsi à la déficience des reins.

La méthode classique en dialyse péritonéale pour éliminer l'excès d'eau (ultrafiltration) et de solutés de l'organisme consiste à injecter dans la cavité péritonéale une solution de dialyse hypertonique par rapport au sang par addition de glucose comme agent osmotique.

Le flux à travers une membrane semi-perméable idéale est principalement déterminé par le nombre total de molécules de soluté (osmolalité) présentes dans la solution, indépendamment de leur taille. En revanche, dans le cas d'une membrane biologique telle que la membrane péritonéale, le flux dépend uniquement des solutés ne traversant pas ou peu la membrane et n'est donc pas nécessairement lié à l'osmolalité totale de la solution.

En outre, la capacité des solutés à traverser la membrane est aussi fonction de la forme des molécules, de leur charge ionique et également de leur taille.

Le choix d'un agent osmotique idéal est délicat : ce dernier doit permettre un gradient osmotique de manière à déplacer l'eau et les substances toxiques du sang vers la solution de dialyse à travers le péritoine. Il doit également être non toxique et biologiquement inerte, tout en étant métabolisable par l'organisme, une partie de celui-ci étant assimilée dans le sang. Il ne doit pas traverser la membrane péritonéale trop rapidement, de manière à maintenir durablement un gradient d'ultrafiltration et ne pas permettre l'accumulation de substances non dégradables, indésirables dans le sang.

Dans son brevet EP 667.356, la société Demanderesse a proposé un procédé de fabrication, à partir d'amidon waxy, d'un hydrolysat d'amidon complètement soluble dans l'eau et de faible indice de polymolécularité, inférieur à 2,8, ayant un Mw compris entre 5.10³ et 1.10⁶ daltons.

Ce procédé consiste à hydrolyser par voie acide un lait d'amidon constitué exclusivement d'amylopectine, puis à compléter cette hydrolyse acide par une hydrolyse enzymatique à l'aide d' α-amylase bactérienne, et à chromatographier cet hydrolysat sur résines cationiques fortes macroporeuses sous forme alcaline ou alcalinoterreuse.

Cet hydrolysat d'amidon particulier, encore appelé icodextrine, présente un poids moléculaire de 12.000 à 20.000 daltons. La majorité des motifs de glucose (plus de 90 %) de l'icodextrine sont liés par des liaisons α-1,4, formant des chaînes linéaires, avec une petite fraction de chaînes α-1,4 branchées en α-1,6 (moins de 10 %) .

L'icodextrine a permis une diminution significative de l'absorption quotidienne de glucose préalablement utilisé comme agent osmotique dans les solutions pour dialyse, amenant ainsi un avantage réel pour le traitement des patients diabétiques et/ou obèses pour lesquels la charge calorique est un facteur critique.

Les solutions de dialyse péritonéale contenant de l'icodextrine comme agent osmotique (notamment commercialisée par BAXTER HEALTHCARE Corp. sous le nom de marque EXTRANEAL^{®}) sont en général utilisées pour les longs échanges quotidiens (nocturnes en dialyse péritonéale continue ambulatoire et diurnes en dialyse péritonéale automatisée).

Cette icodextrine pourrait toutefois être encore améliorée si l'on disposait d'un agent osmotique moins glycémiant, et dont le pouvoir osmotique durerait plus longtemps, amenant un allègement significatif de la procédure du traitement de dialyse.

En effet, le rendement des dialysats étant amélioré, la fréquence de changement des poches de dialyse serait diminuée, ce qui constituerait une amélioration certaine de la qualité de vie du patient.

Ainsi, le glucide idéal en dialyse péritonéale devrait :
être soluble dans l'eau,
avoir une faible viscosité,
ne pas rétrograder, c'est-à-dire ne pas former un gel par réorganisation des macromolécules constitutives dudit glucide,
induire une cinétique lente d'apparition de glucose dans la circulation systémique,
être hydrolyse lentement, mais être complètement dégradé par les enzymes de l'organisme au terme de cette hydrolyse,
exercer une pression osmotique durable.

En effet, en rapport avec ces deux derniers points, le devenir des agents osmotiques administrés en solution dans la cavité péritonéale chez des insuffisants rénaux est déterminé par leur stabilité dans le liquide péritonéal, l'importance de leur absorption dans la circulation sanguine et leur vitesse d'hydrolyse par l'amylase. Or, les agents osmotiques de l'art antérieur présentent tous l'inconvénient d'être hydrolysés trop rapidement.

De tout ce qui précède, il résulte qu'il y a donc un besoin non satisfait de disposer de polymères de glucose présentant des propriétés remarquables, notamment en termes de stabilité et de solubilité, et qui confèrent aux produits qui les contiennent une durée de vie plus longue et une digestibilité contrôlée, ce qui permet de les utiliser en particulier dans les domaines de la dialyse péritonéale, de la nutrition entérale ou parentérale, comme régulateur de la glycémie.

La société Demanderesse a eu le mérite de concilier tous ces objectifs réputés jusqu'alors difficilement conciliables en imaginant et élaborant, au prix de nombreuses recherches, de nouveaux polymères solubles de glucose hautement branchés, obtenus par traitement enzymatique.

Les polymères solubles de glucose hautement branchés conformes à l'invention, ayant une teneur en sucres réducteurs inférieure à 3,5 %, de préférence inférieure à 2,5 %, plus préférentiellement encore inférieure à 1 % sont ainsi caractérisés par le fait qu'ils possèdent un taux de liaisons glucosidiques α-1,6 tout à fait particulier, i.e. compris entre 20 et 30 %, pour une masse moléculaire moyenne en poids choisie dans une fourchette étroite, comprise entre 20.000 et moins de 30.000 daltons.

Ces polymères présentent une osmolalité, déterminée selon un test A (décrit dans EP 1 369 432) d'une valeur inférieure à 25 mOsm/kg, de préférence comprise entre 5 et 20 mOsm/kg.

Comme indiqué ci-dessus, les polymères solubles de glucose branchés conformes à l'invention présentent une faible teneur en sucres réducteurs, inférieure à 3,5 %, de préférence inférieure à 2,5 %, plus préférentiellement encore inférieure à 1 %. Cette teneur en sucres réducteurs peut être adaptée à l'intérieur de ces fourchettes en fonction des besoins liés à l'utilisation. Par exemple, pour l'application en dialyse intrapéritonéale, il est possible de proposer des produits présentant une teneur en sucres réducteurs jusqu'à 3,5 %.

La détermination de la teneur en sucres réducteurs des polymères de glucose branchés conformes à l'invention peut être effectuée par toute méthode connue de l'homme du métier, notamment par des méthodes de réduction des liqueurs cuprotartriques ou par des méthodes colorimétriques à l'acide dinitrosalicylique.

Le taux de liaisons glucosidiques α-1,6 des polymères solubles de glucose branchés conformes à l'invention est déterminé par analyse RMN du proton. Le taux de branchement est alors exprimé en pourcent, correspondant à la quantité de signal du proton, porté par le C1 d'un motif anhydroglucose lié à un autre motif anhydroglucose par une liaison α-1,6, lorsque l'on a donné une valeur de 100 à l'ensemble des signaux des protons glycosidiques portés par tous les C1 des résidus glucose desdits polymères solubles de glucose.

Dans ces conditions, les polymères de glucose solubles hautement branchés conformes à l'invention présentent un taux de liaisons α-1,6 compris entre 20 % et 30 %.

Cette teneur en liaisons glucosidiques α-1,6 confère aux polymères de glucose hautement branchés conformes à l'invention une structure particulière, en termes de degré de ramification par rapport à l'amidon ou au dérivé d'amidon dont ils sont issus.

Cette teneur particulièrement élevée en liaisons glucosidiques α-1,6 rend les polymères de glucose hautement branchés selon l'invention difficilement digestibles, ce qui contribue à pouvoir les utiliser en nutrition entérale comme agent régulateur de la digestion et comme agent régulateur de la glycémie, ou dans toutes les applications où l'on recherche une digestion ralentie apportant du glucose, notamment chez les diabétiques, chez les sportifs ou chez les personnes âgées.

Ils peuvent donc être utilement proposés aux diabétiques ou aux sujets prédisposés, comme aliments, boissons ou adjuvants de nutrition ayant pour fonction d'inhiber l'élévation de la glycémie.

Les masses moléculaires moyennes en poids (Mw) des polymères de glucose de la présente invention sont mesurées par chromatographie d'exclusion stérique (SEC, *size exclusion chromatography*) sur colonnes PSS SUPREMA 100 et PSS SUPREMA 1000 montées en série et couplées à un détecteur de diffusion de la lumière.

Les polymères de glucose solubles hautement branchés de l'invention présentent masse moléculaire moyenne en poids (Mw) comprise entre 20.000 et moins de 30.000 daltons, de préférence comprise entre 20.000 et 28.000, et de manière particulièrement préférée entre 24.000 et 27.000.

Ces polymères de glucose branchés conformes à l'invention constituent une nouvelle famille de polymères, caractérisés par un poids moléculaire particulier, bien différent de celui des polymères solubles de glucose branchés que la société Demanderesse a déjà décrits dans sa demande de brevet EP 1.369.432.

Les polymères solubles de glucose branchés conformes à l'invention présentent également une osmolalité particulièrement faible.

La mesure de l'osmolalité des polymères solubles de glucose branchés conformes à l'invention est réalisée selon le même test A que celui décrit dans la demande de brevet EP 1.369.432, et donne une valeur d'osmolalité inférieure à 25 mOsm/kg, de préférence comprise entre 5 et 20 mOsm/kg.

Il n'existe pas, à la connaissance de la société Demanderesse, de polymères de glucose qui possèdent une telle valeur d'osmolalité, pour un taux de branchement et une masse moléculaire moyenne en poids tels que indiqués ci-dessus.

D'autres mesures, effectuées sur des dextrines limites obtenues par traitement d'amidon liquéfié à l'α-amylase, commercialisées sous le nom BLD 8 par SANMATSU, présentent pour un poids moléculaire compris entre 40.000 et 50.000 daltons et un taux de branchement α-1,6 compris entre 8 et 9 %, une valeur d'osmolalité de plus de 35 mOsm/kg.

Les polymères solubles de glucose de la présente invention sont produits par traitement enzymatique d'amidon. Lors de leur fabrication, ils ne subissent en particulier pas de traitements susceptibles d'introduire des liaisons glucosidiques atypiques telles que les liaisons α- et β-1,2, α- et β-1,3, β-1,4 et β-1,6.

Quant aux polymères solubles de glucose branchés décrits par la société Demanderesse dans sa demande de brevet EP 1.369.432, ils présentent certes une valeur d'osmolalité qui ne dépasse pas les 15 mOsm/kg, mais ont une masse moléculaire moyenne en poids supérieure à celle des polymères de la présente invention, comprise entre 35.000 et 200.000 daltons.

Cette valeur d'osmolalité inférieure à 25 mOsm/kg, de préférence comprise entre 5 et 20 mOsm/kg, confère ainsi aux polymères solubles hautement branchés conformes à l'invention des propriétés qui leur permettent d'être avantageusement utilisés dans le cadre de la nutrition entérale et parentérale.

Leur faible osmolalité et leur profil de masse moléculaire font de ces polymères de glucose hautement branchés conformes à l'invention de parfaits candidats d'agents osmotiques pour des applications en dialyse péritonéale, comme il sera exemplifié ci-après.

La société Demanderesse a en outre constaté que les polymères solubles de glucose hautement branchés conformes à l'invention présentent une meilleure résistance à l'alpha amylase que les polymères solubles de glucose hautement branchés de sa précédente demande de brevet EP 1.369.432. Cette résistance confère aux polymères de glucose des avantages significatifs par rapport aux polymères de l'art antérieur, tels que l'icodextrine. Elle rend ces polymères moins glycémiants et prolonge la durée de leur pouvoir osmotique dans un milieu contenant de l'alpha-amylase, autorisant ainsi leur usage dans les traitements de dialyse de longue durée.

Enfin les polymères solubles de glucose hautement branchés conformes à l'invention peuvent être avantageusement modifiés par voie chimique ou biologique pour accroître leur stabilité, en particulier leur stabilité thermique.

C'est ainsi que ces polymères peuvent être hydrogénés pour fournir des polymères dont l'extrémité finale glucose réductrice est remplacée par une extrémité sorbitol non réductrice.

Une telle transformation peut être obtenue notamment par des techniques d'hydrogénation catalytique bien connues de l'homme de l'art. Ces techniques consistent par exemple à soumettre une solution d'un sucre réducteur, en l'occurrence une solution de polymères solubles de glucose hautement branchés de l'invention, en présence d'un catalyseur au nickel de Raney, à une pression d'hydrogène de 40 à 70 kg/cm² et à une température de 100 à 150°C. L'hydrogénation est poursuivie quelques heures jusqu'à ce que le produit hydrogéné ne montre pratiquement plus de pouvoir réducteur.

Les polymères solubles de glucose hautement branchés selon l'invention peuvent être également soumis à l'action d'enzymes extraites notamment de bactéries du genre Rhizobium, Arthrobacter ou Sulfolobus qui ont la particularité d'isomériser le chaînon maltose réducteur terminal des polymères en chaînon α-α tréhalose non réducteur.

Une telle isomérisation peut être obtenue par exemple grâce aux techniques décrites dans le brevet EP 606.753 dont les enseignements sont incorporés ici par référence. Elle consiste à soumettre une solution de polymères solubles de glucose hautement branchés à l'action d'une enzyme formant des saccharides non réducteurs à une température de 40 à 55°C et un pH compris entre 5 et 10.

Il faut noter ici qu'aucun de ces deux procédés, hydrogénation ou isomérisation enzymatique, ne modifie sensiblement les produits soumis à ces actions.

On peut préparer les polymères solubles de glucose branchés de la présente invention, selon un procédé comprenant la succession des étapes suivantes .
1) préparation d'une solution aqueuse d'amidon waxy, présentant une teneur en matière sèche de 10 à 30 % en poids,
2) traitement de ladite solution successivement avec 2 à 3 ml d'une enzyme de branchement à 30.000 - 50.000 U/ml pour 100 g d'amidon waxy, à une température comprise entre 60 °C et 80 °C pendant une durée de 18 à 24 heures, puis avec une enzyme de saccharification choisie parmi l'amyloglucosidase ou l'α-amylase, de préférence l'amyloglucosidase,
3) fractionnement de la solution obtenue de manière à éliminer les fractions de bas poids moléculaire, de préférence celles ayant une masse moléculaire inférieure à 9000, et récupérer les autres fractions, c'est-à-dire celles de haut poids moléculaire,
4) recueillement des polymères de glucose hautement branchés ainsi obtenus.

L'amidon utilisé dans le procédé revendiqué est un amidon waxy, autrement dit un amidon riche en amylopectine.

Il est toutefois possible d'obtenir les polymères solubles de glucose de la présente invention en appliquant le procédé ci-dessus à des amidons différents des amidons waxy. De manière générale, on peut choisir ceux-ci parmi l'amidon naturel ou hybride issu de pomme de terre, de pomme de terre à haute teneur en amylopectine (fécule waxy), de pois, de riz, de manioc, de blé, de maïs, de maïs ou de blé riches en amylopectine (maïs ou blé waxy), de maïs à haute teneur en amylose, de coupes ou de fractions qui peuvent être faites ou obtenues à partir d'amidons, telles que l'amylose, l'amylopectine, les coupes granulométriques connues de l'homme de l'art comme amidon de blé « A » et amidon de blé « B », et les mélanges des produits susmentionnés.

Les dérivés d'amidon peuvent s'entendre des amidons modifiés issus de la modification enzymatique, chimique et/ou physique, en une ou plusieurs étapes, de cet amidon.

Les dérivés d'amidon peuvent notamment être les amidons modifiés par l'une au moins des techniques connues d'éthérification, d'estérification, de réticulation, d'oxydation, de traitement alcalin, d'hydrolyse acide et/ou enzymatique (à l'origine des maltodextrines et dextrines).

La société Demanderesse a trouvé que les polymères de glucose hautement branchés conformes à l'invention sont aisément synthétisables à partir d'amidons, ou de leurs dérivés, qui présentent déjà un taux de branchement au moins égal à 1 %.

La préparation des polymères de glucose hautement branchés conformes à l'invention est réalisée en modifiant les conditions opératoires déjà décrites dans la demande de brevet EP 1.269.432 de la société Demanderesse.

Dans la demande brevet EP 1.369.432, la société Demanderesse recommandait d'utiliser de 50.000 à 500.000 U d'enzyme de branchement purifiée pour 100 g sec d'amidon ou de dérivé d'amidon, à une température comprise entre 25 et 95°C, de préférence à une température comprise entre 70 et 95°C, pendant une durée de 18 à 24 heures.

Par enzyme de branchement, on entend les enzymes de branchement choisies dans le groupe constitué par les enzymes de branchement du glycogène, les enzymes de branchement de l'amidon et les mélanges quelconques de ces enzymes.

Pour l'obtention des nouveaux polymères de glucose hautement branchés conformes à l'invention, la société Demanderesse recommande de traiter préférentiellement la solution d'amidon, de préférence d'amidon waxy, avec de 2 à 3 ml d'enzyme de branchement à 30.000 - 50.000 U/ml pour 100 g d'amidon, à une température comprise entre 60 et 80°C pendant une durée de 18 à 24 heures.

La deuxième étape du procédé conforme à l'invention consiste également à faire agir une enzyme de saccharification comme l'amyloglucosidase ou l'α-amylase, de préférence l'amyloglucosidase, sur la solution d'amidon waxy préalablement traitée avec l'enzyme de branchement.

Pour l'amyloglucosidase, par exemple, les conditions réactionnelles (température et pH) sont les suivantes : de 0,15 à 0,25 ml d'amyloglucosidase, par exemple de type DEXTROZYME W de NOVOZYMES à 270 AGU/g ou de type OPTIDEX de SOLVAY ENZYMES à 300 AGU/g pour 100 g d'amidon à une température de 60 °C, un pH de 4 à 5, pendant 1 à 3 heures, de préférence pendant 2 heures.

Au terme de ce traitement, les polymères de glucose hautement branchés solubles de l'invention sont obtenus en mélange avec leurs produits de dégradation enzymatiques, majoritairement constitués de glucose, de maltose et d'isomaltose.

La troisième étape du procédé consiste à effectuer un fractionnement à l'aide d'une technique connue choisie par exemple dans le groupe des séparations sur membrane et des chromatographies, de manière à récupérer les fractions de haut poids moléculaire et à éliminer les fractions de bas poids moléculaire, comme décrit dans la demande de brevet EP 1.369.432 de la société Demanderesse.

Quelle que soit la méthode mise en oeuvre, les profils obtenus permettent la séparation de la fraction polysaccharidique de haut poids moléculaire correspondant aux polymères de glucose hautement branchés solubles conformes à l'invention, d'avec les fractions oligosaccharidiques de bas poids moléculaire produits par l'hydrolyse et ayant une masse moléculaire inférieure à 1000 daltons, constituées pour l'essentiel de glucose, de maltose et d'isomaltose.

La dernière étape du procédé conforme à l'invention consiste donc à collecter les fractions de poids moléculaire comprises entre 20.000 et moins de 30.000 daltons correspondant aux polymères de glucose hautement branchés.

Ces fractions peuvent être rassemblées telles quelles, les polymères peuvent être précipités par addition d'éthanol, purifiés et séchés sous vide ou encore par atomisation, par toute technique connue de l'homme du métier.

Les caractéristiques physico-chimiques particulières des polymères de la présente invention permettent leurs applications dans les industries de l'Alimentation, de la Pharmacie, de la Cosmétologie et de la Dermatologie, et plus particulièrement encore dans la Pharmacie, dans les domaines de la nutrition entérale et parentérale, du contrôle de la glycémie, dans le domaine de la dialyse péritonéale en tant qu'agent osmotique et comme substitut de plasma sanguin.

La présente invention a donc également pour objet des compositions, contenant les polymères de glucose hautement branchés décrits ci-dessus. Ces compositions sont notamment destinées à être utilisées chez l'homme ou l'animal dans des applications alimentaires et pharmaceutiques.

Cette composition peut se présenter sous forme solide pour préparation extemporanée, sous forme liquide, par exemple en solution aqueuse ou sous forme concentrée destinée à être diluée avec de l'eau ou avec tout autre diluant adapté.

L'invention a également pour objet l'utilisation de telles compositions dans la nutrition entérale et parentérale et en tant qu'agent régulateur de la glycémie.

En ce qui concerne le domaine particulier de la dialyse péritonéale, la société Demanderesse a trouvé, à l'aide d'un test de résistance à l'alpha-amylase, que les polymères conformes à l'invention sont particulièrement adaptés à la préparation de solutions pour dialyse péritonéale, ainsi qu'il est exemplifié par la suite, où ces polymères sont utilisés comme agent osmotique.

Il est à noter que la société Demanderesse a ainsi vaincu un préjugé technique selon lequel, comme il est affirmé notamment dans la demande de brevet internationale WO 2004/022602, les produits à base d'amidon qui peuvent être utilisés en dialyse péritonéale doivent présenter un degré de ramification α-1,6 préférentiellement compris entre 11 et 18 % et un poids moléculaire compris entre 10.000 et 200.000.

L'invention a également pour objet une solution pour dialyse péritonéale caractérisée en ce qu'elle comprend, en tant qu'agent osmotique, au moins un polymère soluble de glucose hautement branché, obtenu par traitement enzymatique d'amidon, ayant
- une teneur en sucres réducteurs inférieure à 3,5 %,
- un taux de liaisons glucosidiques α-1,6 compris entre 20 et 30 %,
- une masse moléculaire moyenne en poids (Mw), déterminée par diffusion de la lumière, comprise entre 20.000 et moins de 30.000 daltons.

Dans une telle solution pour dialyse péritonéale, le polymère soluble de glucose hautement branché ayant une teneur en sucres réducteurs inférieure à 3,5 %, présente de préférence
- un taux de liaisons glucosidiques α-1,6 compris entre 20 et 25 %
- une masse moléculaire moyenne en poids (Mw), déterminée par diffusion de la lumière d'une valeur comprise entre 24.000 et 27.000 daltons.

La solution pour dialyse péritonéale selon l'invention peut en outre comprendre des électrolytes physiologiquement acceptables, comme le sodium, le potassium, le calcium, le magnésium, le chlore, de manière à éviter la perte par transfert d'électrolytes du sérum vers le péritoine.

La solution lorsqu'elle est obtenue par dissolution dans l'eau des polymères hautement branchés selon l'invention doit être limpide et incolore. Elle est de préférence exempte d'endotoxines, de peptido-glucans et de bêta-glucans, ainsi que de contaminants provenant de la matière première ou des préparations enzymatiques utilisées pour sa fabrication.

A cet effet, les polymères hautement branchés mis en oeuvre dans ladite solution auront de préférence subi une purification de manière à ôter toute coloration ou tout contaminant indésirable tel que protéines, bactéries, toxines bactériennes, fibres, traces de métaux etc.

Cette étape de purification peut être réalisée selon les techniques connues de l'homme du métier.

La solution pour dialyse selon l'invention peut comprendre également des agents tampons (lactate, acétate, gluconate notamment) et autres additifs tels que des aminoacides, de l'insuline, des polyols tels que par exemple le sorbitol, l'érythritol, le mannitol, le maltitol, le xylitol ou les hydrolysats d'amidon hydrogénés.

L'ajout de polyols à la composition, et de préférence de polyols apyrogènes et exempts des impuretés décrites précédemment (endotoxines et autres résidus d'origine bactérienne notamment) permet d'augmenter l'osmolalité de la solution de manière plus avantageuse qu'avec le glucose ou le maltose, en raison de leur pouvoir osmotique supérieur et parce qu'ils ne sont pas réducteurs.

Il est enfin également possible de compléter la solution de dialyse contenant les polymères solubles de glucose hautement branchés de l'invention avec du glucose, du maltose et/ou des polymères de glucose.

Des solutions pour dialyse péritonéale à base d'acides aminés présentent un intérêt certain dans la prévention du vieillissement accéléré du péritoine lié au glucose et à ses dérivés, même si leur coût et le risque d'un délicat contrôle de l'acidose en limite la prescription exclusive et continue.

Il est alors envisageable de composer un mélange de différentes molécules dans une même solution pour dialyse péritonéale : glucose, polymères solubles de glucose hautement branchés conformes à l'invention et acides aminés.

On préfère alors procéder à la stérilisation séparée de ces différents constituants (utilisation de poches compartimentées), afin d'éviter notamment de générer des produits de dégradation du glucose (Glucose Dégradation Products ou GDPs) ou des produits résultants de la liaison du glucose auxdits acides aminés (ou AGEs) responsables d'effets néfastes sur la stabilité de la membrane péritonéale.

La solution pour dialyse selon l'invention est par ailleurs avantageuse par rapport aux produits de l'art antérieur puisque l'agent osmotique qu'elle contient permet d'exercer une pression osmotique durable et induit une cinétique lente d'apparition de glucose, tout en étant stable à la rétrogradation, répondant ainsi aux principaux critères définis précédemment.

D'autres caractéristiques et avantages de l'invention apparaîtront à la lecture des exemples non limitatifs décrits ci-dessous.

### Exemple 1

On prépare un lait d'amidon à partir d'un amidon de maïs waxy fluidifié acide avec un niveau de fluidification WF d'environ 90, commercialisé par la société Demanderesse sous le nom de marque CLEARGUM^{®} CB 90.

Pour cela, on prépare une suspension d'amidon à 25 % de matière sèche sous agitation en ajustant le pH à 7.5.

La solubilisation totale de l'amidon est réalisée dans un cuiseur continu à 145 °C pendant 3 à 4 minutes. Ensuite, la solution est refroidie à 70°C et l'enzyme de branchement du glycogène purifiée de *Bacillus stearothermophilus* (selon un protocole connu par ailleurs de l'homme du métier) est ajoutée en continu à raison de 2.5 ml de solution d'enzyme à 40.000 U/ml pour 100 g sec de substrat.

La réaction enzymatique est menée pendant 20 heures à 70°C et à pH 7 puis stoppée par chauffage à 90°C pendant 1 h.

Le traitement complémentaire avec 0,20 ml d'amyloglucosidase (DEXTROZYME W de NOVOZYMES à 270 AGU/g) pour 100 g sec d'amidon s'effectue dans le milieu réactionnel précédent amené à la température de 60°C et au pH de 4.3.

L'incubation est réalisée pendant 2 heures, et la réaction est stoppée par chauffage 1 h à 90°C.

La solution est alors purifiée par traitement sur charbon actif à raison de 2 % com/sec de NORIT SX+, à pH 5 durant 1 h puis filtrée.

La solution finale est alors fractionnée par ultrafiltration sur membrane avec un seuil de coupure de 9000 daltons (membrane ES209 de PCI), et le rétentat est collecté et atomisé.

Le tableau I suivant présente les résultats des caractéristiques physico-chimiques du polymère soluble de glucose branché conforme à l'invention ainsi obtenu.

**Tableau I**

| | |
|---|---|
| Taux de liaisons α-1,6 (%) | 22.9 |
| Mw (10³ daltons) | 25 |
| Teneur en sucres réducteurs (dosage Bertrand(%)) | 2.3 |
| Glucose (%) | 0.6 |
| Osmolalité (mOsm/kg) | 18 |

### Exemple 2

Cet exemple fait référence à un essai réalisé dans des conditions semi-industrielles.

Dans une cuve de 1.5 m³, 175 kg de CLEARGUM® CB 90 sont délayés dans de l'eau pour atteindre une teneur en matière sèche de 13 %. Le pH est ajusté à 7.

La solubilisation totale de l'amidon est réalisée dans un cuiseur continu semi-industriel à environ 150°C pendant quelques minutes à un débit de 400 kg/h. La solution est alors refroidie en continu à 70°C et l'enzyme de branchement du glycogène purifiée de *Bacillus stearothermophilus* est ajoutée en continu à raison de 2.5 ml de solution d'enzyme à 40.000 U/ml pour 100 g sec de substrat.

La réaction enzymatique est menée pendant 20 heures à 70°C et à pH 7 dans une cuve agitée puis stoppée par chauffage à 90°C pendant 1 h.

Le traitement complémentaire avec 0,18 ml de amyloglucosidase (OPTIDEX de SOLVAY ENZYMES à 300 AGU/g) pour 100 g sec d'amidon s'effectue dans le milieu réactionnel précédent amené à la température de 60°C et au pH de 4.3.

L'incubation est réalisée pendant 2 heures, et la réaction est stoppée par chauffage 1 h à 90°C.

La solution est alors purifiée par traitement à l'aide de charbon actif à raison de 2 % com/sec de NORIT SX+, à pH 5 durant 1 h puis filtrée.

La solution finale est alors fractionnée par ultrafiltration sur membrane avec un seuil de coupure de 9000 daltons (membrane ES209 de PCI).

La solution issue du traitement d'ultrafiltration est alors purifiée par déminéralisation puis par un nouveau traitement par charbon actif à raison de 2 % com/sec de NORIT SX+.

La solution finale est ensuite filtrée puis atomisée dans des conditions standard connues de l'homme du métier.

Le tableau I suivant présente les résultats des caractéristiques physico-chimiques du polymère soluble de glucose branché conforme à l'invention ainsi obtenu.

**Tableau I**

| | |
|---|---|
| Taux de liaisons α-1,6 (%) | 22 |
| Mw (10³ daltons) | 29,8 |
| Teneur en sucres réducteurs dosage Bertrand (%) | 0.6 |
| Glucose (%) | < 0.01 |
| Osmolalité (mOsm/kg) | 8 |

### Exemple 3

On prépare des solutions aqueuses de polymères hautement branchés conformes à l'invention obtenus selon l'exemple 1, que l'on met en contact d'une amylase d'origine pancréatique afin de simuler l'hydrolyse de produits au sein du péritoine.

On suit le déroulement dans le temps de l'hydrolyse amylasique en mesurant les sucres réducteurs formés et le glucose qui apparaît dans le milieu réactionnel.

Ce test permet d'évaluer la résistance des polymères à l'hydrolyse amylasique, qui est un critère essentiel dans le choix d'un agent osmotique pour solution de dialyse.

Le polymère de l'exemple 1 est testé en comparaison avec l'icodextrine (agent osmotique de l'art antérieur).

D'autres polymères, préparés par la société Demanderesse selon l'enseignement de son brevet EP 1.369.432 sont également testés à titre comparatif.

Il s'agit des Produits « a » et « b » tels que préparés conformément à l'exemple 3 et du produit Z tel que préparé conformément à l'exemple 2 de ladite demande de brevet EP 1.369.432.

L'icodextrine est fabriquée conformément au brevet EP 667.356 cité dans la description.

Un témoin maltose est réalisé pour valider le modèle *in vitro* de digestion enzymatique.

Les conditions opératoires pour la digestion amylasique sont les suivantes :
- Peser précisément 0,6 g de produit à tester,
- Ajouter 150 mL de tampon maléate de Na pH 7 à 0,1 mol/L,
- Agiter jusqu'à la dissolution du produit,
- Placer les flacons au bain-marie pendant 15 minutes, pour que la température de la solution soit de 37°C,
- Réaliser un prélèvement de 1,5 ml au temps 0 minute (prélèvement SI),
- Ajouter 0,15 g de pancréatine de porc (α-amylase d'origine animale),
- Incuber à 37°C au bain thermostaté sous agitation pendant 300 minutes,
- Réaliser des prélèvements de 1,5 mL aux temps : 15, 30, 45, 60, 90, 120, 180, 240, 300 minutes,
- Arrêter la réaction enzymatique en plaçant les prélèvements dans un bain à sec à 100°C, pendant 10 minutes,
- Doser le glucose sur les prélèvements, pour simuler l'impact sur la glycémie du produit étudié,
- Doser les sucres réducteurs sur les prélèvements pour étudier la vitesse d'hydrolyse.

Pour le dosage du glucose, on utilise une méthode colorimétrique, réalisée sur automate HITACHI 704 (ROCHE). Le réactif utilisé est un réactif contenant les enzymes GOD/PAP (glucose oxydase, péroxydase).

Le volume de réactif utilisé est de 500 microlitres, le volume d'échantillon est de 5 microlitres et la température de la réaction est de 30°C.

La méthode utilisée pour le dosage des sucres réducteurs est la méthode de SOMOGYI NELSON. Dans un tube bouché, on introduit 200 microlitres d'échantillon, on ajoute 200 microlitres de solution de travail (réactifs tartrate de sodium et sulfate de cuivre).

On porte à ébullition, on ajoute après refroidissement du réactif arsénomolybdique, puis de l'eau.

La solution obtenue est déposée dans une microplaque, puis on lit l'absorbance au lecteur de microplaques à une longueur d'onde de 520 nanomètres.

Les résultats sont rassemblés dans les tableaux suivantes :

**1. cinétique d'apparition du glucose (en % libéré sur sec)**

| Temps (min) | MALTOSE | Polymère selon l'invention | "a" | "b" | Z | ICODEXTRINE |
|---|---|---|---|---|---|---|
| SI | 0.26 | 0,36 | 3,35 | 0,00 | 0,53 | 0,28 |
| 15 | 0.79 | 1,79 | 5,31 | 1,59 | - | 3,07 |
| 30 | 1.06 | 1,91 | 5, 68 | 1,96 | 2,11 | 3, 63 |
| 45 | 1.59 | 2,21 | 5,86 | 2,24 | - | 3,91 |
| 60 | 2.12 | 2,12 | 6,14 | 2,52 | 2,37 | 4,19 |
| 90 | 2.65 | 2,43 | 6,52 | 2,89 | - | 4,75 |
| 120 | 3.44 | 2,63 | 6,61 | 3,17 | 2,90 | 5,31 |
| 180 | 5.03 | 3,21 | 7,26 | 4,76 | 3,43 | 6,15 |
| 240 | 6.35 | 3,62 | 8,10 | - | 3,96 | 6,99 |
| 300 | 7.68 | 4,01 | 8,38 | 5,41 | 4,22 | 7,82 |

**2. cinétique d'apparition des sucres réducteurs (en % sur sec)**

| Temps (min) | MALTOSE | Polymère selon l'invention | "a" | "b" | Z | ICODEXTRINE |
|---|---|---|---|---|---|---|
| SI | 51,01 | 2,25 | 5,76 | 0,88 | 1,45 | 2,74 |
| 15 | 47, 94 | 10,20 | 19,33 | 18,96 | - | 30,39 |
| 30 | 48,29 | 9,53 | 20,00 | 19,04 | 11,00 | 32,53 |
| 45 | 48,55 | 11, 93 | 20,25 | 19,78 | - | 32,46 |
| 60 | 48,84 | 11,42 | 19,92 | 20,80 | 11,10 | 32,95 |
| 90 | 49,42 | 11,29 | 20,37 | 19, 42 | - | 34,16 |
| 120 | 47,15 | 10,23 | 21, 68 | 21,04 | 12,16 | 34,40 |
| 180 | 48,87 | 13,04 | 22,46 | 21,79 | 12,22 | 36, 64 |
| 240 | 50,90 | 12,60 | 23,05 | 23,11 | 12,29 | 37,03 |
| 300 | 52,20 | 13,66 | 22,67 | 22,88 | 13,64 | 37,06 |

**3. synthèse des résultats**

| PRODUITS TESTES | % de glucose libéré à 300 min. | % de sucres réducteurs formés à 300 min. | Taux de liaisons α-1,6 en % | Masse Molaire (daltons) |
|---|---|---|---|---|
| MALTOSE | 7, 68 | 52,20 | 0 | 342 |
| ICODEXTRINE | 7,82 | 37,06 | 6,5 - 8 | 12.000 - 20.000 |
| Polymère conforme à l'invention | 4,01 | 13, 66 | 22,9 | 25.000 |
| "a" | 8,38 | 22,67 | 19,4 | 33000 |
| "b" | 5,41 | 22,88 | 14,3 | 68000 |
| Z | 4,22 | 13,64 | 24,2 | 45000 |

D'après les résultats obtenus avec les composés « a », « b » et Z, la société Demanderesse, dans sa demande de brevet EP 1.369.432 en déduisait déjà que plus le taux de branchement (le taux de liaisons α-1,6) augmente, plus l'hydrolyse amylasique est diminuée. Cette dernière est également dépendante du poids moléculaire. Ainsi, plus le taux de branchement est élevé et le poids moléculaire petit, moins la molécule est attaquée par l'amylase.

Si le polymère soluble de glucose branché selon l'invention confirme cette tendance, il faut cependant noter que bien que présentant un taux de branchement α-1,6 inférieur au composé Z, et un poids moléculaire pratiquement 2 fois plus faible, il se caractérise par un taux d'hydrolyse équivalent, voire légèrement inférieur, à celui dudit composé Z.

Le polymère soluble de glucose branché de l'invention constitue donc le meilleur compromis en termes de poids moléculaire, structure branchée et comportement quant à sa résistance amylasique.

Par ailleurs, par rapport à l'icodextrine qui est un composé de référence en tant qu'agent osmotique en dialyse péritonéale, les polymères solubles de glucose conformes à l'invention en constituent une variante améliorée car ils présentent une résistance à l'hydrolyse par l'amylase pancréatique nettement supérieure à celle l'icodextrine.

Cela signifie surtout que le produit de la présente invention présente un avantage certain en termes de pouvoir glycémiant, pour un poids moléculaire très voisin de l'icodextrine.

Pour confirmer cette observation, la société Demanderesse a entrepris une étude visant à comparer la capacité de ces deux composés à exercer un pouvoir osmotique durable dans le temps, par la détermination de la répartition des masses molaires du produit après action de l'a-amylase pancréatique.

Il est en effet bien connu de l'homme du métier que plus un composé est hydrolyse rapidement en petites molécules, moins il pourra exercer un pouvoir osmotique durable dans le temps en dialyse péritonéale.

Le tableau suivant traduit la répartition des masses molaires de l'icodextrine et du polymère soluble de glucose branché conforme à l'invention, masses molaires mesurées après digestion par l' α-amylase. Cette répartition est exprimée en % de produits dont le poids moléculaire est supérieur à 1.000 daltons.

| | % de masses molaires > 1000 daltons |
|---|---|
| ICODEXTRINE | 37,8 |
| Polymère conforme à l'invention | 88 |

Il apparaît donc qu'environ 62 % de l'icodextrine ont été hydrolysés en molécules de moins de 1.000 daltons après 300 min de digestion amylasique, alors que seuls 22 % l'ont été pour le polymère soluble de glucose conforme à l'invention.

A poids moléculaire équivalent, le polymère soluble de glucose branché conforme à l'invention présente donc un bien meilleur comportement que l'icodextrine, et constitue un produit très intéressant pour une utilisation en dialyse péritonéale.

## Revendications

1. Polymères solubles de glucose hautement branchés, obtenus par traitement enzymatique d'amidon, ayant une teneur en sucres réducteurs inférieure à 3,5 %, de préférence inférieure à 2,5 %, plus préférentiellement encore inférieure à 1 %, **caractérisés en ce qu'**ils présentent :
- un taux de liaisons glucosidiques α-1,6 compris entre 20 et 30 %,
- une masse moléculaire moyenne en poids (Mw), déterminée par diffusion de la lumière, comprise entre 20.000 et moins de 30.000 daltons.

2. Polymères selon la revendication 1, **caractérisés en ce qu'**ils présentent une osmolalité, déterminée selon un test A, d'une valeur inférieure à 25 mOsm/kg, de préférence comprise entre 5 et 20 mOsm/kg, le test A consistant à déterminer l'osmolalité d'une solution renfermant 100 g sec desdits polymères de glucose hautement branchés placés dans 1 kg d'eau, à l'aide d'un osmomètre MARK 3 de FISKE®ASSOCIATES.

3. Polymères selon la revendication 1 ou 2, **caractérisés en ce qu'**ils sont isomérisés enzymatiquement de manière à ce que leurs extrémités réductrices soient transformées en tréhalose, ou qu'ils sont hydrogénés.

4. Procédé de préparation des polymères solubles de glucose hautement branchés selon l'une quelconque des revendications 1 à 3, **caractérisé par le fait que** l'on :
1) prépare une solution aqueuse d'amidon waxy, présentant une teneur en matière sèche de 10 à 30 % en poids,
2) traite successivement ladite solution avec 2 à 3 ml d'une enzyme de branchement à 30.000 - 50.000 U/ml pour 100 g d'amidon waxy, à une température comprise entre 60 °C et 80 °C pendant une durée de 18 à 24 heures, puis avec une enzyme de saccharification choisie parmi l'amyloglucosidase ou l'α-amylase, de préférence l'amyloglucosidase,
3) effectue un fractionnement de la solution obtenue de manière à éliminer les fractions de bas poids moléculaire et récupérer les fractions de haut poids moléculaire,
4) recueille les polymères de glucose hautement branchés ainsi obtenus.

5. Procédé de préparation de polymères solubles de glucose hautement branchés selon la revendication 4, **caractérisé par le fait que** l'on traite la solution aqueuse d'amidon :
- avec de 2 à 3 ml d'enzyme de branchement à 30.000 - 50.000 U/ml pour 100 g d'amidon, à une température comprise entre 60 et 80 °C pendant une durée de 18 à 24 heures,
- puis avec de 0,15 à 0,25 ml d'amyloglucosidase pour 100 g d'amidon, à une température de 60 °C, à un pH de 4 à 5, pendant une durée comprise entre 1 et 3 heures, de préférence pendant 2 heures.

6. Compositions destinées à être utilisées chez l'homme et l'animal dans des applications alimentaires et pharmaceutiques, **caractérisées en ce qu'**elles contiennent les polymères de glucose hautement branchés selon l'une quelconque des revendications 1 à 3 ou susceptibles d'être obtenus par le procédé selon l'une ou l'autre des revendications 4 et 5.

7. Utilisation des compositions selon la revendication 6, dans la nutrition entérale et parentérale et comme agent régulateur de la glycémie.

8. Solution pour dialyse péritonéale, **caractérisée en ce qu'**elle comprend, en tant qu'agent osmotique, au moins un polymère soluble hautement branché selon l'une quelconque des revendications 1 à 3.

9. Solution pour dialyse selon la revendication 8, **caractérisée en ce que** le polymère soluble hautement branché ayant une teneur en sucres réducteurs inférieure à 3,5 % présente :
- un taux de liaisons glucosidiques α-1,6 compris entre 20 et 25 %
- une masse moléculaire moyenne en poids (Mw), déterminée par diffusion de la lumière, comprise entre 24.000 et 27.000 daltons.

10. Solution pour dialyse péritonéale selon la revendication 8 ou 9, **caractérisée en ce qu'**elle comprend en outre un polyol choisi dans le groupe formé par le sorbitol, le mannitol, le maltitol, le xylitol, l'érythritol et les hydrolysats d'amidon hydrogénés.

11. Solution pour dialyse péritonéale selon la revendication 10, **caractérisée en ce qu'**elle comprend en outre des agents tampons tels que les sels de lactate, d'acétate et de gluconate.

12. Solution pour dialyse péritonéale selon l'une quelconque des revendications 8 à 11, **caractérisée en ce qu'**elle comprend en outre du glucose, du maltose et/ou des polymères de glucose.

13. Solution pour dialyse péritonéale selon l'une quelconque des revendications 8 à 11, **caractérisée en ce qu'**elle comprend en outre des acides aminés et du glucose.

## Patentansprüche

1. Lösliche, hochverzweigte Glucosepolymere, welche durch eine Enzymbehandlung von Stärke erhalten werden, und einen Gehalt an reduzierenden Zuckern kleiner 3,5%, bevorzugt kleiner 2,5%, stärker bevorzugt kleiner 2,5% und noch stärker bevorzugt kleiner 1% aufweisen, **dadurch gekennzeichnet, dass** sie aufweisen:
- einen Anteil an α-1,6 Glukosidbindungen von 20 bis 30%,
- ein durch Lichtstreuung bestimmtes gewichtsgemitteltes Molekulargewicht (Mw) zwischen 20.000 und weniger als 30.000 Daltons.

2. Polymere nach Anspruch 1, **dadurch gekennzeichnet, dass** sie eine anhand eines Tests A ermittelte Osmolalität mit einem Wert kleiner 25 mOsm/kg, vorzugsweise zwischen 5 und 20 mOsm/kg aufweisen, wobei der Test A darin besteht, mittels eines Osmometers MARK 3 von FISKE®ASSOCIATES die Osmolalität einer Lösung zu bestimmen, welche 100 g Trockengewicht der hochverzweigten Glucosepolymere in 1 kg Wasser umfasst.

3. Polymere nach Anspruch 1 oder 2, **dadurch gekennzeichnet, dass** sie enzymatisch isomerisiert sind, so dass ihre reduzierenden Enden in Trehalose umgewandelt oder hydriert werden.

4. Zubereitungsverfahren für lösliche, hochverzweigte Glucosepolymere nach einem der Ansprüche 1 bis 3, **dadurch gekennzeichnet, dass**:
1) eine wässrige Lösung einer Wachsstärke zubereitet wird, welche einen Trockenmassegehalt von 10 bis 30 Gewichtsprozent aufweist,
2) diese Lösung aufeinanderfolgend bei einer Temperatur zwischen 60 °C und 80 °C während eines Zeitraums von 18 bis 24 Stunden mit 2 bis 3 ml eines Verzweigungsenzyms mit 30.000 - 50.000 U/ml auf 100g Wachsstärke behandelt wird, dann mit einem Verzuckerungsenzym, gewählt aus Amyloglucosidase oder 1'α-Amylase, bevorzugt Amyloglucosidase,
3) eine Fraktionierung der erhaltenen Lösung durchgeführt wird, um die Fraktionen mit geringem Molekulargewicht zu eliminieren und die Fraktionen mit hohem Molekulargewicht zu gewinnen,
4) die so erhaltenen hochverzweigten Glucosepolymere gesammelt werden.

5. Zubereitungsverfahren für lösliche, hochverzweigte Glucosepolymere nach Anspruch 4, **dadurch gekennzeichnet, dass** die wässrige Stärkelösung behandelt wird:
- mit 2 bis 3 ml Verzweigungsenzym mit 30.000 - 50.000 U/ml auf 100g Stärke bei einer Temperatur zwischen 60 und 80 °C während eines Zeitraums von 18 bis 24 Stunden,
- dann mit 0,15 bis 0,25 ml Amyloglucosidase auf 100 g Stärke bei einer Temperatur von 60 °C und einem pH-Wert von 4 bis 5, während eines Zeitraums von 1 bis 3 Stunden, vorzugsweise während 2 Stunden.

6. Zusammensetzungen, welche zur Verwendung beim Menschen und beim Tier in Nahrungsmittel- und pharmazeutischen Anwendungen bestimmt sind, **dadurch gekennzeichnet, dass** diese die hochverzweigten Glucosepolymere nach einem der Ansprüche 1 bis 3 umfassen oder welche durch ein Verfahren nach einem der Ansprüche 4 und 5 erhalten werden können.

7. Anwendung der Zusammensetzungen nach Anspruch 6 in der enteralen und parenteralen Ernährung und als blutzuckerregulierendes Mittel.

8. Lösung für die peritoneale Dialyse, **dadurch gekennzeichnet, dass** sie als osmotisches Mittel wenigstens ein lösliches hochverzweigtes Glucosepolymer nach einem der Ansprüche 1 bis 3 enthält.

9. Dialyselösung nach Anspruch 8, **dadurch gekennzeichnet, dass** das lösliche hochverzweigte Glucosepolymer mit einem Gehalt an reduzierenden Zuckern kleiner 3,5% folgendes aufweist:
- einen Anteil an α-1,6 Glukosidbindungen von 20 bis 25%,
- ein durch Lichtstreuung bestimmtes gewichtsgemitteltes Molekulargewicht (Mw) zwischen 24.000 und 27.000 Daltons.

10. Lösung für die peritoneale Dialyse nach Anspruch 8 oder 9, **dadurch gekennzeichnet, dass** diese ferner ein Polyol aufweist, welches aus der Gruppe, umfassend Sorbitol, Mannitol, Maltitol, Xylitol, Erythritol und Hydrolysate von hydrierter Stärke gewählt ist.

11. Lösung für die peritoneale Dialyse nach Anspruch 10, **dadurch gekennzeichnet, dass** diese ferner Puffersubstanzen umfasst, wie beispielsweise Lactat-, Acetat- und Gluconatsalze.

12. Lösung für die peritoneale Dialyse nach einem der Ansprüche 8 bis 11, **dadurch gekennzeichnet, dass** diese ferner Glucose, Maltose und/oder Glucosepolymere aufweist.

13. Lösung für die peritoneale Dialyse nach einem der Ansprüche 8 bis 11, **dadurch gekennzeichnet, dass** diese ferner Aminosäuren und Glucose aufweist.

## Claims

1. A soluble highly branched glucose polymer which is obtained by enzymatic treatment of starch, having a reducing sugars content of less than 3.5%, preferably less than 2.5%, even more preferably less than 1%, **characterized in that** it exhibits:
- a proportion of α-1,6-glucosidic linkages of between 20% and 30%,
- a weight-average molecular weight (Mw), determined by light scattering, of between 20.000 and less than 30.000 daltons.

2. The polymer as claimed in claim 1, **characterized in that** it exhibits an osmolality, determined according to a test A, having a value of less than 25 mOsm/kg, preferably between 5 and 20 Osm/kg, the test A consists in determining the osmolality of a solution containing 100 g on a dry basis of highly branched glucose polymer placed in 1 kg of water, with a FISKE^{®}ASSOCIATES MARK 3 osmometer.

3. The polymer as claimed in claims 1 or 2, **characterized in that** it is enzymatically isomerized in such a way that its reducing ends are converted to trehalose, or **in that** it is hydrogenated.

4. A process for preparing the soluble highly branched glucose polymer as claimed in claims 1 to 3, **characterized in that**:
1) an aqueous solution of waxy starch, having a solids content of from 10% to 30% by weight, is prepared,
2) said solution is treated successively with 2 to 3 ml of a branching enzyme at 30.000-50.000 U/ml per 100 g of waxy starch, at a temperature of between 60 °C and 80 °C for a period of 18 to 24 hours, and then with a saccharification enzyme chosen from amyloglucosidase or α-amylase, preferably amyloglucosidase,
3) the solution obtained is fractionated so as to remove the low-molecular-weight fractions and to recover the high-molecular-weight fractions,
4) the highly branched glucose polymers thus obtained are recovered.

5. The process for preparing soluble highly branched glucose polymers as claimed in claim 4, **characterized in that** the aqueous starch solution is treated:
- with from 2 to 3 ml of branching enzyme at 30.000-50.000 U/ml per 100 g of starch, at a temperature of between 60 and 80 °C for a period of 18 to 24 hours,
- then with from 0.15 to 0.25 ml of amyloglucosidase per 100 g of starch, at a temperature of 60 °C, at a pH of 4 to 5, for a period of between 1 and 3 hours, preferably for 2 hours.

6. Compositions intended to be used in humans and animals in food and pharmaceutical applications, **characterized in that** it contains the highly branched glucose polymer as claimed in claims 1 to 3, or obtainable by the process as claimed in any one of claims 4 or 5.

7. The use of the compositions as claimed in claim 6, in enteral and parenteral nutrition and as a glycemia regulating agent.

8. A solution for peritoneal dialysis, **characterized in that** it comprises, as osmotic agent, at least one soluble highly branched polymer as claimed in any one of claims 1 to 3.

9. The solution for dialysis as claimed in claim 8, **characterized in that** the soluble highly branched polymer having a reducing sugars content of less than 3.5% exhibits:
- a proportion of α-1,6-glucosidic linkages of between 20% and 25%,
- a weight-average molecular weight (Mw), determined by light scattering, of between 24.000 and 27.000 daltons.

10. The solution for peritoneal dialysis as claimed in claims 8 or 9, **characterized in that** it also comprises a polyol chosen from the group formed by sorbitol, mannitol, maltitol, xylitol, erythritol and hydrogenated starch hydrolysates.

11. The solution for peritoneal dialysis as claimed in claim 10, **characterized in that** it also comprises buffering agents such as lactate salts, acetate salts and gluconate salts.

12. The solution for peritoneal dialysis as claimed in any one of claims 8 to 11, **characterized in that** it also comprises glucose, maltose and/or glucose polymers.

13. The solution for peritoneal dialysis as claimed in any one of claims 8 to 11, **characterized in that** it also comprises amino acids and glucose.
